(19) Europäisches Patentamt
European Patent Office
Office européen des brevets

(11) Veröffentlichungsnummer: **0 446 740 A2**

# (12) EUROPÄISCHE PATENTANMELDUNG

(21) Anmeldenummer: **91103173.0**

(22) Anmeldetag: **02.03.91**

(51) Int. Cl.[5]: **C07C 69/712**, C07C 67/08, C08K 5/10

(30) Priorität: **12.03.90 DE 4007765**

(43) Veröffentlichungstag der Anmeldung: **18.09.91 Patentblatt 91/38**

(84) Benannte Vertragsstaaten: **BE CH DE FR GB IT LI NL**

(71) Anmelder: **BASF Aktiengesellschaft**
**Carl-Bosch-Strasse 38**
**W-6700 Ludwigshafen(DE)**

(72) Erfinder: **Neumann, Peter, Dr.**
**Poststrasse 28**
**W-6800 Mannheim 31(DE)**
Erfinder: **Trauth, Hubert**
**Milanstrasse 6**
**W-6724 Dudenhofen(DE)**
Erfinder: **Aumueller, Alexander, Dr.**
**An der Marlach 5**
**W-6705 Deidesheim(DE)**

## (54) Verwendung von Benzophenon-Derivaten als Lichtschutzmittel für aromatische Polycarbonate und aromatische Polyestercarbonate.

(57) Verwendung von Benzophenon-Derivaten I

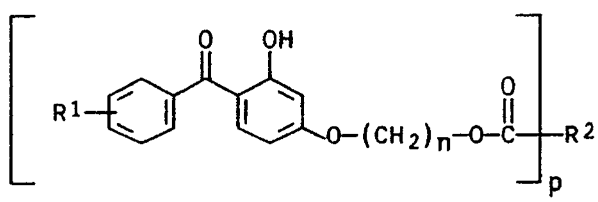

I

bei denen die Variablen die folgende Bedeutung haben:

$R^1$ Wasserstoff, $C_1$-$C_4$-Alkyl, $C_1$-$C_4$-Alkoxy, Phenoxy, Fluor, Chlor, Brom, Cyano oder Trifluormethyl,
n 2 bis 4,
p 1 bis 4 und
$R^2$
(a) für den Fall p = 1
$C_1$-$C_{12}$-Alkyl, das durch eine oder mehrere Hydroxyl- oder $C_1$-$C_4$-Alkoxygruppen substituiert sein kann,
Cycloalkyl mit 3 bis 6 C-Atcmen im Ring,
ein Rest der allgemeinen Formel

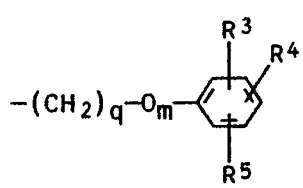

oder

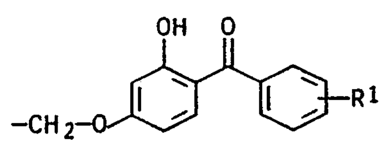

wobei $R^3$, $R^4$ und $R^5$ Wasserstoff, $C_1$-$C_4$-Alkyl oder $C_1$-$C_4$-Alkoxy oder einer oder zwei der Reste $R^3$

EP 0 446 740 A2

bis $R^5$ Fluor, Chlor, Brom, Cyano oder Trifluormethyl oder einer der Reste $R^3$ bis $R^5$ Phenyl oder Phenoxy bedeuten, q einen Wert von 0 bis 4 hat und m für 0 oder 1 steht, oder
ein fünf- oder sechsgliedriger ungesättigter heterocyclischer Ring, der durch $C_1$-$C_4$-Alkylgruppen, Fluor, Chlor, Brom oder Cyano substituiert sein kann, oder

(b) für den Fall p = 2 bis 4
eine Einfachbindung oder die Ergänzung zu einer p-wertigen aliphatischen, cycloaliphatischen, aromatischen oder heterocyclischen Carbonsäure

als Lichtschutzmittel für aromatische Polycarbonate und aromatische Polyestercarbonate.

Die vorliegende Erfindung betrifft die Verwendung von Benzophenon-Derivaten der allgemeinen Formel I

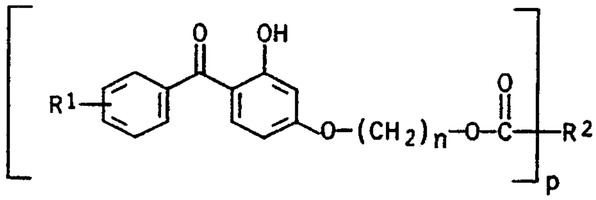

I

in der die Variablen die folgende Bedeutung haben:

R1 Wasserstoff, $C_1$-$C_4$-Alkyl, $C_1$-$C_4$-Alkoxy, Phenoxy, Fluor, Chlor, Brom, Cyano oder Trifluormethyl,

n 2 bis 4,

p 1 bis 4 und

$R^2$

(a) für den Fall p = 1

$C_1$-$C_{12}$-Alkyl, das durch eine oder mehrere Hydroxyl- oder $C_1$-$C_4$-Alkoxygruppen substituiert sein kann,

Cycloalkyl mit 3 bis 6 C-Atomen im Ring,

ein Rest der allgemeinen Formel

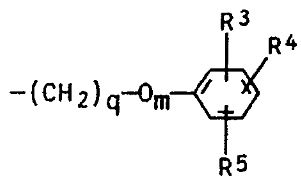

oder

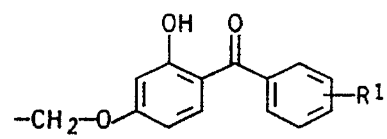

wobei $R^3$, $R^4$ und $R^5$ Wasserstoff, $C_1$-$C_4$-Alkyl oder $C_1$-$C_4$-Alkoxy oder einer oder zwei der Reste $R^3$ bis $R^5$ Fluor, Chlor, Brom, Cyano oder Trifluormethyl oder einer der Reste $R^3$ bis $R^5$ Phenyl oder Phenoxy bedeuten, q einen Wert von 0 bis 4 hat und m für 0 oder 1 steht, oder ein fünf- oder sechsgliedriger ungesättigter heterocyclischer Ring, der durch $C_1$-$C_4$-Alkylgruppen, Fluor, Chlor, Brom oder Cyano substituiert sein kann, oder

(b) für den Fall p = 2 bis 4

eine Einfachbindung oder die Ergänzung zu einer p-wertigen aliphatischen, cycloaliphatischen, aromatischen oder heterocyclischen Carbonsäure

als Lichtschutzmittel für aromatische Polycarbonate und aromatische Polyestercarbonate.

Außerdem betrifft die Erfindung gegen die Einwirkung von Licht stabilisierte aromatische Polycarbonate und aromatische Polyestercarbonate.

Die Verbindungen I sind zum Teil neue Stoffe. Deshalb betrifft die Erfindung weiterhin diese neuen Verbindungen sowie ein Verfahren zu ihrer Herstellung.

Aus der US-A 3 676 471 (1) sind verschiedene Benzophenon-Derivate I bekannt. Diese Substanzen werden als Lichtschutzmittel für polymere Zusammensetzungen wie Polyolefine, Polyvinylchlorid und seine Copolymerisate mit anderen ungesättigten Verbindungen, Polystyrol und seine Copolymerisate mit Butadien, Acrylnitril oder Methacrylsäureestern, Polyester, Polyamide sowie Lacke aus synthetischen Harzen empfohlen.

Die EP-A 254 987 (2) und 309 909 (3) betreffen jeweils ein Verfahren zum Färben von textilem Polyestermaterial mit Dispersionsfarbstoffen, bei dem im Falle von (2) ein Teil der unter (a) für p = 1 bezeichneten Benzophenon-Derivate I und im Falle von (3) die unter (b) für p = 2 bis 4 bezeichneten Benzophenon-Derivate I Anwendung finden.

Die in der Literatur für Polycarbonate und Polyestercarbonate bereits beschriebenen Lichtschutzmittel, beispielsweise Benztriazol-Derivate, wie sie aus der EP-B 006 564 (4) bekannt sind, weisen eine Reihe von Nachteilen auf. Mit ihnen wird kein zufriedenstellender Schutz gegen die Zerstörung durch Lichteinwirkung unter atmosphärischen Bedingungen erzielt, so daß diese Kunststoffe zu rasch altern. Insbesondere wird durch den Zusatz dieser Mittel häufig bei Polycarbonatartikeln wie transparenten Platten und Profilen eine

Verfärbung hervorgerufen, diese Artikel erscheinen dann in der Durchsicht gelb statt farblos.

Aufgabe der vorliegenden Erfindung war es deshalb, Mittel bereitzustellen, die die beschriebenen Nachteile nicht aufweisen.

Demgemäß wurden die eingangs definierten Benzophenon-Derivate I gefunden. Als Alkyl- und Alkoxyreste $R^1$ seien beispielsweise genannt: Methyl, Ethyl, n-Propyl, iso-Propyl, n-Butyl, tert.-Butyl, Methoxy, Ethoxy, n-Propyloxy, iso-Propyloxy, n-Butyloxy und tert.-Butyloxy. Die Reste $R^1$ können in o-, m- oder p-Stellung stehen.

Weiterhin stehen die Reste $R^1$ in erster Linie für Wasserstoff, Methyl, Methoxy, Phenoxy und Chlor, insbesondere wird Wasserstoff bevorzugt.

Die Anzahl n der Methylengruppen beträgt vorzugsweise 2.

Als Reste $R^2$ für den Fall (a) mit p = 1 seien beispielhaft genannt:

- Methyl, Ethyl, n-Propyl, iso-Propyl, n-Butyl, iso-Butyl, sek.-Butyl, tert.-Butyl, n-Pentyl, 3-Methylbutyl, 1-Ethylpropyl, 1,1-Dimethylpropyl, 2,2-Dimethylpropyl, n-Hexyl, n-Heptyl, 1-Ethylpentyl, n-Octyl, 2, 4, 4-Trimethylpentyl, 2-Ethylhexyl, n-Nonyl, n-Decyl, n-Undecyl, n-Dodecyl, Hydroxymethyl, 2-Hydroxyethyl, 2-Hydroxypropyl, 2-Hydroxy-2-methylpropyl, 3-Hydroxy-2-methylpropyl, 2-Hydroxybutyl, Methoxymethyl und 2-Methoxyethyl; bevorzugt werden $C_1$-$C_4$-Alkylreste
- Cyclopropyl, Cyclobutyl, Cyclopentyl sowie vorzugsweise Cyclohexyl
- Phenyl, 2-, 3- und 4-Methylphenyl, 2-, 3- und 4-Ethylphenyl, 2-, 3-und 4- n- oder iso-Propylphenyl, 2-, 3- und 4- n- oder tert.-Butylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2, 4, 6-Trimethylphenyl, 2,4-Dimethyl-6-tert.-butylphenyl, 2-, 3- und 4-Chlorphenyl, 2-, 3- und 4-Bromphenyl, 2,4-Dichlorphenyl, 2,5-Dichlorphenyl, 2,6-Dichlorphenyl, 3,5-Dichlorphenyl, 2-, 3- und 4-Methoxyphenyl, 2,3-Dimethoxyphenyl, 2,4-Dimethoxyphenyl, 2,6-Dimethoxyphenyl, 3,4-Dimethoxyphenyl, 3,5-Dimethoxyphenyl, 2-Ethoxyphenyl, 4-Ethoxyphenyl, 2,3,4-Trimethoxyphenyl, 3,4,5-Trimethoxyphenyl, 4-Biphenyl, 4-Phenoxyphenyl, Benzyl, 2-, 3-und 4-Methylbenzyl, 4-Ethylbenzyl, 4-Isopropylbenzyl, 4-tert.-Butylbenzyl, 2-, 3- und 4-Chlorbenzyl, 2-, 3- und 4-Brombenzyl, 2-, 3- und 4-Methoxybenzyl, 2-, 3- und 4-Ethoxybenzyl, 3,4-Dimethoxybenzyl, 3,5-Dimethoxybenzyl, 3,4,5-Trimethoxybenzyl, 2-Phenylethyl, 3-Phenylpropyl, 4-Phenylbutyl, Phenoxymethyl, 2-, 3- und 4-Methylphenoxymethyl, 2-, 3- und 4-Ethylphenoxymethyl, 4-Isopropylphenoxymethyl, 4-n-Butylphenoxymethyl, 4-tert.-Butylphenoxymethyl, 2,3-Dimethylphenoxymethyl, 2,4-Dimethylphenoxymethyl, 2,5-Dimethylphenoxymethyl, 3,5-Dimethylphenyoxymethyl, 3,4-Dimethylphenoxymethyl, 2-Methyl-4-tert.-butylphenoxymethyl, 3,4,5-Trimethoxyphenoxymethyl, 2,4,6-Trimethylphenoxymethyl, 2,6-Dimethyl-4-tert.-butyl-phenoxymethyl, 2-, 3- und 4-Chlorphenoxymethyl, 2-, 3- und 4-Bromphenoxymethyl, 2-, 3- und 4-Methoxyphenoxymethyl, 4-Ethoxyphenoxymethyl, 2-Methyl-4-chlorphenoxylmethyl, 2-Phenoxyethyl und 4-Phenoxybutyl; bevorzugt werden Phenyl, Methylphenyl-Reste (Tolyl-Reste), Dimethylphenyl-Reste (Xylyl-Reste), tert.-Butylphenyl-Reste, Methoxyphenyl-Reste, Dimethoxyphenyl-Reste, Benzyl, 2-Phenylethyl, Phenoxymethyl und 2-Phenoxyethyl
- Reste der allgemeinen Formel

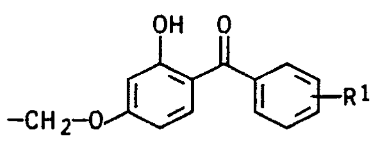

  wobei $R^1$ bevorzugt Wasserstoff, Methyl, Methoxy, Phenoxy oder Chlor, insbesondere Wasserstoff, bezeichnet
- als heterocyclische Ringe Furyl-2, Furyl-3, 2,5-Dimethylfuryl-3, Thienyl-2, Thienyl-3, Pyridyl-2, Pyridyl-3, Pyridyl-4 und 2-Chlorpyridyl-3.

Für den Fall (b) mit p = 2 bis 4 seien als p-wertige Carbonsäuren, aus denen durch Weglassen der Carboxylgruppen die Ergänzungen $R^2$ abgeleitet werden, beispielhaft genannt:

Oxalsäure, Malonsäure, Bernsteinsäure, Glutarsäure, 3,3-Dimethylglutarsäure, Adipinsäure, Pimelinsäure, Korksäure, Azelainsäure, Sebacinsäure, Maleinsäure, Fumarsäure, Glutaconsäure, Itaconsäure, Citronensäure, Tricarballylsäure, Butan-1,2,3,4-tetracarbonsäure, Ethylendiamintetraessigsäure, Phthalsäure, Isophthalsäure, Terephthalsäure, Hexahydrophthalsäure, Hexahydroisophthalsäure, Hexahydroterephthalsäure, 3- und 4-Chlorphthalsäure, Tetrachlorphthalsäure, m-Phenylendiessigsäure, p-Phenylendiessigsäure, m-Phenylendioxiessigsäure, p-Phenylendioxiessigsäure, Diphenylether-4,4'-dicarbonsäure, Diphensäure, Diphenyl-4,4'-dicarbonsäure, Diphenylsulfon-4,4'-dicarbonsäure, Benzophenon-4,4'-dicarbonsäure, Naphthalin-2,6-dicarbonsäure, Trimellitsäure, Trimesinsäure, Pyromellitsäure, Hemimellitsäure, Thiophen-2,5-dicarbonsäure, Pyridin-2,6-, Pyridin-2,5- und Pyridin-3,5-dicarbonsäure.

Die Ergänzung $R^2$ steht für eine Einfachbindung, wenn man ihn von Oxalsäure ableitet (p = 2).

Für den Fall (b) beträgt der Wert für p vorzugsweise 2 oder 3, insbesondere 2.

Die Erfindung betrifft weiterhin neue Benzophenon-Derivate der allgemeinen Formel Ia

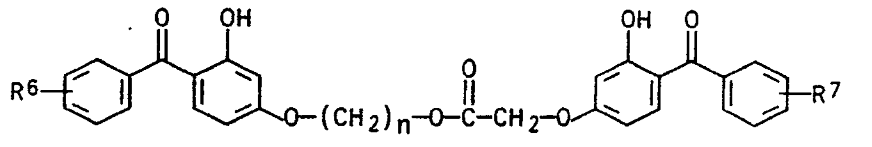

Ia

in der die Variablen die folgende Bedeutung haben:

$R^6$,$R^7$ Wasserstoff, $C_1$-$C_4$-Alkyl, $C_1$-$C_4$-Alkoxy, Phenoxy, Fluor, Chlor, Brom, Cyano oder Trifluormethyl und

n 2 bis 4.

Die Verbindungen Ia stellen eine Untergruppe der Verbindungen I dar.

Die Benzophenon-Derivate Ia werden zweckmäßigerweise durch Veresterung eines Alkohols der allgemeinen Formel II

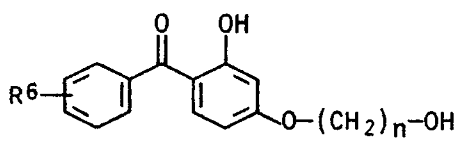

II

mit einer Carbonsäure der allgemeinen Formel III

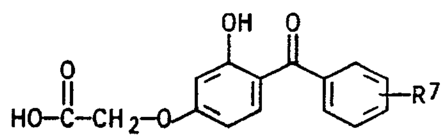

III

in an sich bekannter Weise hergestellt.

Man arbeitet hierbei in der Regel in einem organischen Lösungsmittel wie Benzol, Toluol oder Xylol, bei Temperaturen von 60 bis 150 °C und in Gegenwart eines sauren Katalysators, beispielsweise Schwefelsäure, p-Toluolsulfonsäure oder eines sauren Ionenaustauscherharzes. Das sich bei der Umsetzung bildende Wasser wird vorteilhaft während der Reaktion kontinuierlich destillativ entfernt.

Die Benzophenon-Derivate I dienen als Lichtschutzmittel vor allem für aromatische Polycarbonate, daneben aber auch für aromatische Polyestercarbonate.

Unter aromatischen Polycarbonaten sind Homopolycarbonate, Copolycarbonate und Terpolycarbonate sowie Mischungen hieraus zu verstehen. Bevorzugte Polycarbonate sind solche mit einem als Gewichtsmittel bestimmten Molekulargewicht von 10 000 bis 200 000, insbesondere 20 000 bis 80 000, und einer Flow Rate by Extrusion Plastometer von 1 bis 24 g/10 min bei 300 °C gemäß Annual Book of ASTM Standards D 1238-86.

Diese Polycarbonate werden nach bekannten Verfahren, beispielsweise aus Phosgen und Dihydroxyverbindungen durch Polykondensation, erhalten. Als Dihydroxyverbindungen kommen z.B. in Betracht:

Hydrochinon, Resorcin, Bis(hydroxyphenyl)alkane, Bis(hydroxyphenyl)cycloalkane, Bis(hydroxyphenyl)-ether, Bis(hydroxyphenyl)ketone, Bis(hydroxyphenyl)sulfoxide, Bis(hydroxyphenyl)sulfone oder 2,2-Bis-(hydroxyphenyl)diisopropylbenzole. Im einzelnen seien genannt: 2,4-Bis(4-hydroxyphenyl)-2-methylbutan, 1,1-Bis(4-hydroxyphenyl)cyclohexan, Bis(4-hydroxyphenyl)-p-diisopropylbenzol, 2,2-Bis(3-chlor-4-hydroxyphenyl)propan, 4,4'-Dihydroxybenzophenon, 4,4'-Dihydroxydiphenylsulfon und insbesondere 2,2-Bis(4-hydroxyphenyl)propan (Bisphenol A).

Als aromatische Polyestercarbonate sind vor allem solche zu nennen, die aus Diphenolen (beispielsweise Bisphenol A), Iso- und/oder Terephthalsäure, Phosgen oder einem anderen Kohlensäurederivat, Kettenabbrechern und gegebenenfalls Vernetzungsmitteln erhalten werden. Sie haben in der Regel

relative Viskositäten, d.h. ein Verhältnis der Viskosität der Lösung zur Viskosität des reinen Lösungsmittels, von 1,2 bis 2,0, gemessen in 0,5 gew.-%iger Lösung in Dichlormethan bei 25° C.

Die erfindungsgemäß zu verwendenden Benzophenon-Derivate I können in Mengen von 0,05 bis 10 Gew.-%, vorzugsweise 0,5 bis 3 Gew.-%, bezogen auf die Menge des Polymeren, eingesetzt werden. Die Einarbeitung in das Polymere erfolgt in der Regel in der Schmelze, beispielsweise in Extrudern.

Außer den Verbindungen I können die organischen Materialien noch weitere Hilfs- und Füllstoffe in den hierfür üblichen Mengen enthalten, beispielsweise:

- weitere Lichtschutzmittel wie 2-(2'-Hydroxyphenyl)benztriazole, 2,4-Bis(2'-hydroxyphenyl)-6-alkyl-s-triazine, 2-Hydroxybenzophenone und 1,3-Bis(2'-hydroxybenzoyl)benzole
- Antioxidantien auf der Basis von Phosphor- und Schwefelverbindungen und von sterisch gehinderten Phenolen und Aminen
- Füllstoffe wie Ruß, Kaolin, Talk und Glasfasern
- Pigmente wie Titandioxid
- Sonstige Zusatzstoffe wie Weichmacher, Gleitmittel, Emulgatoren, optische Aufheller, Flammschutzmittel und Antistatica.

Bei Verwendung der genannten Antioxidantien kann die stabilisierende Wirkung gegen die Einwirkung von Licht und auch gegen thermische Belastung noch deutlich gesteigert werden. Diese Mittel oder Gemische davon werden zweckmäßigerweise in einer Menge von 0,1 bis 5 Gew.%, vorzugsweise 0,5 bis 3 Gew.%, bezogen auf die Menge des Polymeren, zusammen mit den Verbindungen I eingesetzt.

Als phosphorhaltige Antioxidantien sind z.B.
Tris(nonylphenyl)phosphit,
Distearylpentaerythritdiphosphit,
Tris(2,4-di-tert.-butylphenyl)phosphit,
Tris(2-tert.-butyl-4-methylphenyl)phosphit,
Bis(2,4-di-tert.-butylphenyl)pentaerythritdiphosphit und
Tetrakis(2,4-di-tert.-butylphenyl)-4,4'-biphenylendiphosphit
zu nennen.

Als schwefelhaltige Antioxidationsmittel sind z.B.
Dilaurylthiodipropionat,
Dimyristylthiodipropionat,
Distearylthiodipropionat,
Pentaerythrittetrakis(β-laurylthiopropionat) und
Pentaerythrittetrakis(β-hexylthiopropionat)
zu nennen.

Als sterisch gehinderte phenolische Antioxidationsmittel kommen z.B.
2,6-Di-tert.-butyl-4-methylphenol,
n-Octadecyl-β-(3,5-di-tert.-butyl-4-hydroxyphenyl)propionat,
1,1,3-Tris(2-methyl-4-hydroxy-5-tert.-butylphenyl)butan,
1,3,5-Trimethyl-2,4,6-tris(3,5-di-tert.-butyl-4-hydroxybenzyl)benzol,
1,3,5-Tris(3,5-di-tert.-butyl-4-hydroxybenzyl)isocyanurat,
1,3,5-Tris[(3,5-di-tert.-butyl-4-hydroxyphenyl)propionyloxy-ethyl]isocyanurat,

1,3,5-Tris(2,6-di-methyl-3-hydroxy-4-tert.-butylbenzyl)isocyanurat,
Pentaerythrit-tetrakis[β-(3,5-di-tert.-butyl-4-hydroxyphenyl)propionat]
Sowie Tocopherol
in Betracht.

Als sterisch gehinderte Amine kommen z.B.
Bis(2,2,6,6-tetramethylpiperidyl)sebacat,
Bis(1,2,2,6,6-pentamethylpiperidyl)sebacat,
das Kondensationsprodukt von 1-Hydroxyethyl-2,2,6,6-tetramethyl-4-hydroxypiperidin und Bernsteinsäure,
das Kondensationsprodukt von N,N'-(2,2,6,6-Tetramethylpiperidyl)hexamethylendiamin und 4-tert.-Octylamino-2,6-dichlor-1,3,5-s-triazin,
Tris(2,2,6,6-tetramethylpiperidyl)nitrilotriacetat,
Tetrakis(2,2,6,6-tetramethyl-4-piperidyl)-1,2,3,4-butan-tetracarbonsäure, 1,1'-(1,2-Ethandiyl)-bis(3,3,5,5-tetramethylpiperazinon) und
die Kondensationsprodukte von 4-Amino-2,2,6,6-tetramethylpiperidinen und Tetramethylol-acetylendiharnstoffen
in Betracht.

Als Vorteile gegenüber den aus dem Stand der Technik bekannten Lichtschutzmitteln seien hervorgehoben, daß die erfindungsgemäß zu verwendenden Verbindungen I eine deutlich geringere Eigenfarbe aufweisen und damit insbesondere bei transparenten Polycarbonat- oder Polyestercarbonatteilen keine Verfärbung mehr hervorrufen. Darüber hinaus zeigen die erfindungsgemäß zu verwendenden Verbindungen I einen deutlich verbesserten Stabilisationseffekt gegenüber der Einwirkung von Licht, d.h. die Alterung des polymeren Materials verläuft in ihrer Gegenwart wesentlich langsamer.

Herstellbeispiele (Nr. 1 bis 20)

Allgemeine Arbeitsvorschrift zur Herstellung von Benzophenon-Derivaten Ia

1,0 mol eines Alkohols II

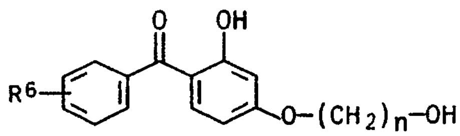

II

und 0,80 mol einer Carbonsäure III

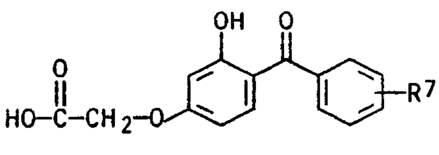

III

wurden zusammen mit 50 g p-Toluolsulfonsäure-monohydrat in 2,5 l Toluol 6 Stunden unter Rückfluß erhitzt. Dabei wurde Wasser kontinuierlich am Wasserabscheider destillativ entfernt. Nach Abkühlung auf Raumtemperatur wurde der entstandene Niederschlag abfiltriert, mit n-Hexan gewaschen und bei 60 °C und 50 bis 100 mbar getrocknet. Zur weiteren Reinigung wurde aus Toluol oder aus einem Alkanol, in einigen Fällen unter Zusatz von Bleicherde, umkristallisiert.

Tabelle 1 zeigt die Einzelheiten und die Ergebnisse dieser Versuche.

Tabelle 1: Herstellung von Benzophenon-Derivaten Ia (n = 2)

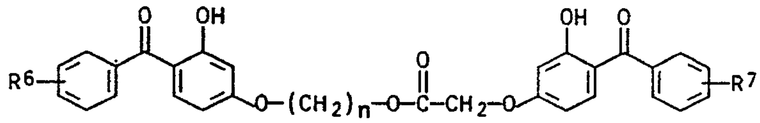

| Beispiel Nr. | $R^6$ | $R^7$ | umkristallisiert aus | Schmelzpunkt [°C] |
|---|---|---|---|---|
| 1 | H | H | Toluol* | 134-136 |
| 2 | H | Cl | n-Propanol | 152-154 |
| 3 | H | $CH_3$ | Isopropanol* | 150-152 |
| 4 | H | $OCH_3$ | n-Propanol | 137-139 |
| 5 | Cl | H | n-Propanol | 153-155 |
| 6 | Cl | Cl | n-Butanol | 176-178 |
| 7 | Cl | $CH_3$ | n-Butanol | 189-191 |
| 8 | Cl | $OCH_3$ | n-Butanol | 158-159 |
| 9 | $CH_3$ | H | n-Propanol | 146-148 |
| 10 | $CH_3$ | Cl | n-Butanol | 171-173 |
| 11 | $CH_3$ | $CH_3$ | n-Propanol | 140-141 |
| 12 | $CH_3$ | $OCH_3$ | n-Propanol | 150-151 |
| 13 | $OCH_3$ | H | n-Butanol | 188-190 |
| 14 | $OCH_3$ | Cl | n-Butanol | 158-161 |
| 15 | $OCH_3$ | $CH_3$ | n-Propanol | 128-130 |
| 16 | $OCH_3$ | $OCH_3$ | n-Butanol | 148-150 |
| 17 | $OC_6H_5$ | H | n-Butanol | 131-133 |
| 18 | $OC_6H_5$ | Cl | n-Butanol | 146-148 |
| 19 | $OC_6H_5$ | $CH_3$ | n-Propanol | 132-134 |
| 20 | $OC_6H_5$ | $OCH_3$ | n-Propanol | 144-146 |

*unter Bleicherde-Zusatz

Anwendungsbeispiele (Nr. 21 bis 28)

Zur Herstellung von Belichtungsproben wurden 2,5 g einer der in Tabelle 2 genannten Verbindungen in 1000 g eines handelsüblichen aromatischen Polycarbonats (Makrolon® Pc 2800 der Firma Bayer) durch einmaliges Extrudieren bei 280 °C eingearbeitet und das anfallende Granulat wurde mit Hilfe einer Spritzgußmaschine bei 300 °C zu 2 mm dicken Prüfkörpern verarbeitet.

Die hergestellten Prüfkörper wurden in einem Xenotest® 200-Schnellbewitterungsgerät der Firma Hanau auf ihre Licht- und Wetterechtheit getestet. Die Alterung wurde durch Messung des Yellowness-Indexes (YI) gemäß Annual Book of ASTM Standards D 1925-70 (Reapproved 1977) nach definierten Zeitintervallen bestimmt. Die maximale Belichtungsdauer betrug bei allen Versuchen 2000 h. Die Ergebnisse sind in Tabelle 2 zusammengestellt.

Tabelle 2: YI-Werte von Polycarbonat-Proben

| Beispiel Nr. | Stabilisator aus Beispiel | YI-Wert nach 0 h | 1000 h | 1500 h | 2000 h |
|---|---|---|---|---|---|
| erfindungsgemäß: | | | | | |
| 21 | 1 | 9,2 | 8,3 | 10,0 | 11,5 |
| 22 | 3 | 12,5 | 12,2 | 14,2 | 16,4 |
| 23 | 5 | 11,1 | 13,4 | 14,3 | 16,2 |
| 24 | 9 | 10,5 | 12,2 | 14,4 | 16,0 |
| 25 | 11 | 9,6 | 13,1 | 14,5 | 16,6 |
| 26 | 14 | 12,9 | 12,5 | 15,8 | 16,3 |
| 27 | 17 | 11,8 | 12,5 | 16,0 | 16,2 |
| 28 | * | 8,1 | 12,1 | 14,1 | 16,8 |
| zum Vergleich: | | | | | |
| ohne Stabilisator | | 10,5 | 17,2 | 18,4 | 22,4 |
| Benztriazol-Derivat** | | 9,0 | 13,0 | 15,0 | 18,2 |

* 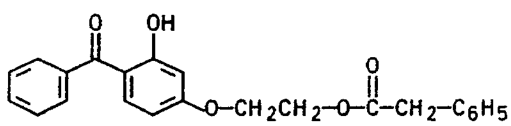 gemäß Literaturstelle (2)

** 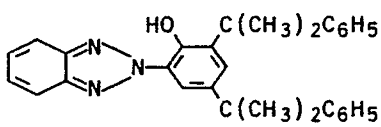 gemäß Literaturstelle (4)

**Patentansprüche**

**1.** Verwendung von Benzophenon-Derivaten der allgemeinen Formel I

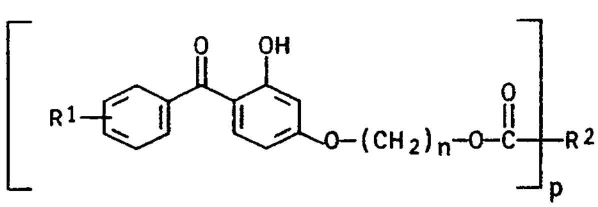

I

in der die Variablen die folgende Bedeutung haben:

$R^1$ Wasserstoff, $C_1$-$C_4$-Alkyl, $C_1$-$C_4$-Alkoxy, Phenoxy, Fluor, Chlor, Brom, Cyano oder Trifluormethyl,

n 2 bis 4,

p 1 bis 4 und

$R^2$

(a) für den Fall p = 1

$C_1$-$C_{12}$-Alkyl, das durch eine oder mehrere Hydroxyl- oder $C_1$-$C_4$-Alkoxygruppen substitu-

iert sein kann,

Cycloalkyl mit 3 bis 6 C-Atomen im Ring,

ein Rest der allgemeinen Formel

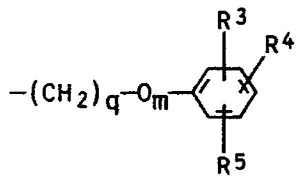

oder

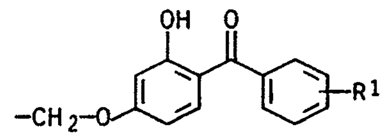

wobei $R^3$, $R^4$ und $R^5$ Wasserstoff, $C_1$-$C_4$-Alkyl oder $C_1$-$C_4$-Alkoxy oder einer oder zwei der Reste $R^3$ bis $R^5$ Fluor, Chlor, Brom, Cyano oder Trifluormethyl oder einer der Reste $R^3$ bis $R^5$ Phenyl oder Phenoxy bedeuten, q einen Wert von 0 bis 4 hat und m für 0 oder 1 steht, oder

ein fünf- oder sechsgliedriger ungesättigter heterocyclischer Ring, der durch $C_1$-$C_4$-Alkylgruppen, Fluor, Chlor, Brom oder Cyano substituiert sein kann, oder

(b) für den Fall p = 2 bis 4

eine Einfachbindung oder die Ergänzung zu einer p-wertigen aliphatischen, cycloaliphatischen, aromatischen oder heterocyclischen Carbonsäure

als Lichtschutzmittel für aromatische Polycarbonate und aromatische Polyestercarbonate.

**2.** Verwendung von Benzophenon-Derivatem I nach Anspruch 1, bei denen die Variablen die folgende Bedeutung haben:

- $R^1$ Wasserstoff, Methyl, Methoxy, Phenoxy oder Chlor,
- n 2,
- p 1 und
- $R^2$ $C_1$-$C_4$-Alkyl, Cyclohexyl, Phenyl, Tolyl, Xylyl, tert.-Butylphenyl, Methoxyphenyl, Dimethoxyphenyl, Benzyl, 2-Phenylethyl, Phenoxymethyl, 2-Phenoxyethyl oder die Gruppe

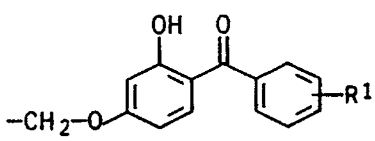

wobei in dieser Formel der Rest $R^1$ die in diesem Anspruch genannte Bedeutung hat.

**3.** Verwendung von Benzophenon-Derivaten I nach Anspruch 1, bei denen die Variablen die folgende Bedeutung haben:

- $R^1$ Wasserstoff, Methyl, Methoxy, Phenoxy oder Chlor,
- n 2,
- p 2 bis 4 und
- $R^2$ eine Einfachbindung oder die Ergänzung zu einer p-wertigen aliphatischen, cycloaliphatischen aromatischen oder heterocyclischen Carbonsäure.

**4.** Benzophenon-Derivate der allgemeinen Formel Ia

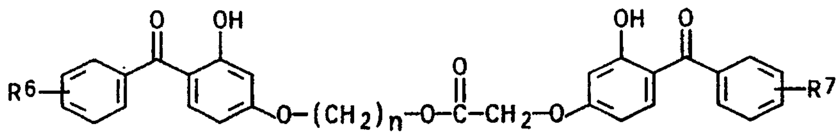

Ia

in der die Variablen die folgende Bedeutung haben:

$R^6$, $R^7$ Wasserstoff, $C_1$-$C_4$-Alkyl, $C_1$-$C_4$-Alkoxy, Phenoxy, Fluor, Chlor, Brom, Cyano oder Trifluormethyl und

n 2 bis 4.

**5.** Verfahren zur Herstellung der Benzophenon-Derivate Ia gemäß Anspruch 4, dadurch gekennzeichnet, daß man einen Alkohol der allgemeinen Formel II

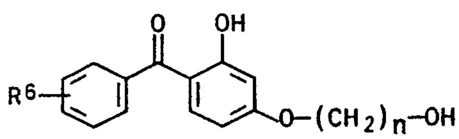

II

mit einer Carbonsäure der allgemeinen Formel III

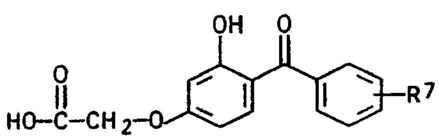

III

in an sich bekannter Weise verestert.

**6.** Gegen die Einwirkung von Licht stabilisierte aromatische Polycarbonate und aromatische Polyestercarbonate, enthaltend 0,05 bis 10 Gew.-%, bezogen auf die Menge des Polymeren, eines Benzophenon-Derivates I gemäß Anpruch 1 oder 2.